# EUROPEAN PATENT APPLICATION

(11) **EP 4 202 045 A1**
(43) Date of publication of application: **28.06.2023**
(21) Application number: 21216959.3
(22) Date of filing: 22.12.2021
(51) Int. Cl.: C12N 15/11, A61K 31/712, A61K 31/7115

(54) **SYNTHETIC TRANSFER RNA WITH MODIFIED NUCLEOTIDES**

(71) Applicant: Universität Hamburg, 20148 Hamburg (DE)
(72) Inventor: Ignatova, Zoya, 20146 Hamburg (DE); Albers, Suki, 22850 Norderstedt (DE)
(74) Representative: Stüven, Ralf

(57) **Abstract**

The invention relates to a synthetic transfer RNA with at least one modified nucleotide or combination of modified nucleotides at a unique position or a unique pattern of modified nucleotides.

## Description

The invention relates to a synthetic transfer RNA with modified nucleotides.

Transfer ribonucleic acids (tRNAs) are an essential part of the protein synthesis machinery of living cells as necessary components for translating the nucleotide sequence of a messenger RNA (mRNA) into the amino acid sequence of a protein. Naturally occurring tRNAs comprise an amino acid binding stem being able to covalently bind an amino acid and an anticodon loop containing a base triplet called "anticodon", which can bind non-covalently to a corresponding base triplet called "codon" on an mRNA. A protein is synthesized by assembling the amino acids carried by tRNAs using the codon sequence on the mRNA as a template with the aid of a multi component system comprising, inter alia, the ribosome and several auxiliary enzymes.

tRNAs have recently seen increasing interest in their use as drugs for therapeutical purposes, e.g. as part of a gene therapy for treating conditions associated with nonsense mutations, i.e. mutations changing a sense codon encoding one of the twenty amino acids specified by the genetic code to a chain-terminating codon ("premature termination codon", PTC) in a gene sequence (Ai-Ming Yu, Young Hee Choi and Mei-Juan Tu, RNA Drugs and RNA Targets for Small Molecules: Principles, Progress, and Challenges, Pharmacological Reviews, 2020, 72 (4) 862-898; DOI: 10.1124/pr. 120.019554; Porter, JJ, Heil, CS, Lueck, JD, Therapeutic promise of engineered nonsense suppressor tRNAs, WIREs RNA. 2021; 12:e1641, DOI: 10.1002/wrna.1641). Lueck et al. (Lueck, J.D., Infield, DT, Mackey, AL, Pope, RM, McCray, PB, Ahern, CA. Engineered tRNA suppression of a CFTR nonsense mutation, bioRxiv 088690; doi: 10.1101/088690), for example, describe a codon-edited tRNA enabling the conversion of an in-frame stop codon in the CFTR gene to the naturally occurring amino acid in order to restore the full-length wild type protein.

Compared to mRNA, tRNA molecules offer significantly higher stability and are on average 10-fold shorter, alleviating the problem of introduction into the target tissue. This has led to attempts to use tRNA in gene therapy in order to prevent the formation of a truncated protein from an mRNA with a premature stop codon and to introduce the correct amino acid instead (see, e.g., Koukuntla, R 2009, Suppressor tRNA mediated gene therapy, Graduate Theses and Dissertations, 10920, Iowa State University, http://lib.dr.iastate.edu/etd/10920; US 2003/0224479 A1; US 6964859).

However, nucleic acids with unmodified nucleotides are inherently immunogenic and a potent activator of the immune response in humans - an evolutionary selected mechanism to recognize foreign DNA/RNA and fight viral and bacterial attacks. In contrast, human DNA and RNA elicits a much lower immune response because of its higher posttranscriptional modifications. Pioneering studies of Kariko and Weissman have shown that, in mRNA, posttranscriptionally modified nucleotides are less immunogenic to the human host. Uracil (U) was identified as eliciting the highest immune response when unmodified, and replacing all uracils with 5m-pseudoU completely eliminated the inherent immunogenicity of mRNA and its ability to elicit an immune response, and also enhanced the translational capacity of the mRNA (Karikó K, Buckstein M, Ni H, Weissman D., 2005, Suppression of RNA recognition by Toll-like receptors: the impact of nucleoside modification and the evolutionary origin of RNA, Immunity 23(2): 165-75, doi: 10.1016/j.immuni.2005.06.008; Karikó K, Weissman D., 2007, Naturally occurring nucleoside modifications suppress the immunostimulatory activity of RNA: implication for therapeutic RNA development, Curr Opin Drug Discov Devel. 10(5):523-32; Karikó, K., Muramatsu, H., Welsh, F. A., Ludwig, J., Kato, H., Akira, S., & Weissman, D. (2008). Incorporation of pseudouridine into mRNA yields superior nonimmunogenic vector with increased translational capacity and biological stability. Mol Ther. 16(11), 1833-1840, doi: 10.1038/mt.2008.200; US 8278036 B2; US 10,232,055 B2). This finding is one of the main reasons for the recent successful application of mRNA vaccines in the coronavirus pandemic (see, for example, Pardi, N., Hogan, M., Porter, F. et al. mRNA vaccines - a new era in vaccinology, Nat Rev Drug Discov 17, 261-279 (2018), doi: 10.1038/nrd.2017.243; Zhang C, Maruggi G, Shan H and Li J (2019), Advances in mRNA Vaccines for Infectious Diseases, Front. Immunol. 10:594. doi: 10.3389/fimmu.2019.00594; Xu S, Yang K, Li R, Zhang L., (2020), mRNA Vaccine Era - Mechanisms, Drug Platform and Clinical Prospection, Int J Mol Sci. 21(18):6582, doi: 10.3390/ijms21186582).

For transfer-RNA, it has been shown that 2'-O-methylation of guanine at position 18 (Gm18) within bacterial tRNA acts as a suppressor of innate immune activation in humans (Rimbach K, Kaiser S, Helm M, Dalpke A, H, Eigenbrod T: 2'-O-Methylation within Bacterial RNA Acts as Suppressor of TLR7/TLR8 Activation in Human Innate Immune Cells, J Innate Immun 2015;7:482-493, doi: 10.1159/000375460; Jöckel, S., Nees, G., Sommer, R., Zhao, Y., Cherkasov, D., Hori, H., Ehm, G., Schnare, M., Nain, M., Kaufmann, A., & Bauer, S. (2012), The 2'-O-methylation status of a single guanosine controls transfer RNA-mediated Toll-like receptor 7 activation or inhibition, The Journal of experimental medicine, 209(2), 235-241, doi: 10.1084/jem.20111075; Kaiser, S., Rimbach, K., Eigenbrod, T., Dalpke, A., Helm, M., (2014), A modified dinucleotide motif specifies tRNA recognition by TLR7, RNA 20, doi: 10. 1261/rna.044024.113).

The general approach described above for mRNA, i.e. to completely replace all uracils with 5m-pseudoU, however, is not applicable to tRNA, because modifying nucleotides may disfavour a tRNA secondary structure which is essential for tRNA function, and may consequently decrease tRNA decoding activity. In addition, in tRNA the majority of the nucleotides are engaged in secondary interactions, e.g. internal base pairing, which decrease/abolish their immunogenicity. Several modifications of tRNA nucleotides are known (see, for example, Suzuki, T. The expanding world of tRNA modifications and their disease relevance, Nat Rev Mol Cell Biol 22, 375-392 (2021). doi:/10.1038/s41580-021-00342-0;), but the diverse effects of these nucleotide modifications in terms of, for example, molecule stability, anticodon specificity, reading frame maintenance or reading activity, are not well understood (Väre, V.Y.P.; Eruysal, E.R.; Narendran, A.; Sarachan, K.L.; Agris, P.F. Chemical and Conformational Diversity of Modified Nucleosides Affects tRNA Structure and Function. Biomolecules 2017, 7, 29. doi:/10.3390/biom7010029; Agris, P.F., 2015, The importance of being modified: an unrealized code to RNA structure and function, RNA 21: 552-554; doi:10.1261/rna.050575.115; Duechler M, Leszczynska G, Sochacka E, Nawrot B. Nucleoside modifications in the regulation of gene expression: focus on tRNA. Cell Mol Life Sci. 2016 Aug;73(16):3075-95. doi:10.1007/s00018-016-2217-y).

It is an object of the invention to provide a tRNA, in particular for therapeutic purposes, that is little or not immunogenic, but still serves its function in the translational machinery of a living cell, preferably a human cell.

For solving the problem, the invention provides, in one aspect, a synthetic transfer ribonucleic acid (RNA) comprising at least one modified nucleotide at a given position or at least one pair of modified nucleotides at given positions, the modified nucleotide or pair of modified nucleotides and their position or positions being selected from the following list:
a) m5C or 2'-OMeC at position 11,
b) m6A, m1A or 2'-OMeA at position 21,
c) Ψ, 2'-OMeΨ, m5U, m1Ψ, or 2'-OMeU at position 37,
d) m5C or 2'-OMeC at position 56,
e) m6A, m1A or 2'-OMeA at position 59,
f) Ψ, 2'-OMeΨ, m5U, m1Ψ, or 2'-OMeU at position 60,
g) m5C, m6A, m5U, m1A, 2'-OMeA, 2'-OMeC, 2'-OMeU, 2'-OMeΨ, Ψ, or m1Ψ at position 73,
h) m6A, m1A or 2'-OMeA at position 49 and Ψ, 2'-OMeΨ, m5U, m1Ψ, or 2'-OMeU at position 65,
i) m6A, m1A or 2'-OMeA at position 65 and Ψ, 2'-OMeΨ, m5U, m1Ψ, or 2'-OMeU at position 49,
j) Ψ, 2'-OMeΨ, m5U, m1Ψ, or 2'-OMeU at position 2 and m6A, m1A or 2'-OMeA at position 71,
k) Ψ, 2'-OMeΨ, m5U, m1Ψ, or 2'-OMeU at position 71 and m6A, m1A or 2'-OMeA at position 2,
l) Ψ, 2'-OMeΨ, m5U, m1Ψ, or 2'-OMeU at position 5 and m6A, m1A or 2'-OMeA at position 68,
m) Ψ, 2'-OMeΨ, m5U, m1Ψ, or 2'-OMeU at position 68 and m6A, m1A or 2'-OMeA at position 5,
n) m6A, m1A or 2'-OMeA at position 29 and Ψ, 2'-OMeΨ, m5U, m1Ψ, or 2'-OMeU at position 41, or
o) m6A, m1A or 2'-OMeA at position 41 and Ψ, 2'-OMeΨ, m5U, m1Ψ, or 2'-OMeU at position 29,
the position numbering following transfer RNA numbering convention.

The invention provides novel tRNAs that differ from known tRNAs, in particular from naturally occurring human tRNAs, in that they contain at least one modified nucleotide or a combination or pattern of two or more modified nucleotides at positions at which known tRNAs do not have the same modified nucleotide or combination of two or more modified nucleotides.

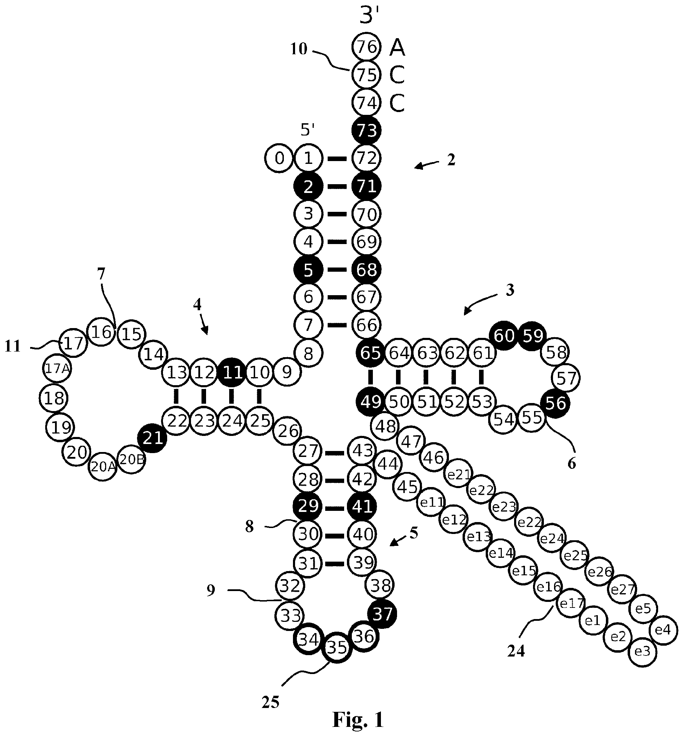

The terms "transfer ribonucleic acid" or "tRNA" refer to RNA molecules with a length of typically 73 to 90 nucleotides, which mediate the translation of a nucleotide sequence in a messenger RNA into the amino acid sequence of a protein. tRNAs are able to covalently bind a specific amino acid at their 3' CCA tail at the end of the acceptor stem, and to base-pair via a usually three-nucleotide anticodon in the anticodon loop of the anticodon arm with a usually three-nucleotide sequence (codon) in the messenger RNA. Some anticodons can pair with more than one codon due to a phenomenon known as wobble base pairing. The secondary "cloverleaf' structure of tRNA comprises the acceptor stem binding the amino acid and three arms ("D arm", "T arm" and "anticodon arm") ending in loops (D loop, T loop (TψC loop), anticodon loop), i.e. sections with unpaired nucleotides. The terms "D stem", "T stem" (or "TψC stem") and "anticodon stem" (also "AC stem") relate to portions of the D arm, T arm and anticodon arm, respectively, with paired nucleotides. Aminoacyl tRNA synthetases charge (aminoacylate) tRNAs with a specific amino acid. Each tRNA contains a distinct anticodon triplet sequence that can base-pair to one or more codons for an amino acid. By convention, the nucleotides of tRNAs are often numbered 1 to 76, starting from the 5'-phosphate terminus, based on a "consensus" tRNA molecule consisting of 76 nucleotides, and regardless of the actual number of nucleotides in the tRNA, which are not always of a length of 76 nt due to variable portions, such as the D loop or the variable loop in the tRNA (see Fig. 1). Following this convention, nucleotide positions 34-36 of naturally occurring tRNA refer to the three nucleotides of the anticodon, and positions 74-76 refer to the terminating CCA tail. Any "supernumerary" nucleotide can be numbered by adding alphabetic characters to the number of the previous nucleotide being part of the consensus tRNA and numbered according to the convention, for example 20a, 20b etc. for the D-arm, or by independently numbering the nucleotides and adding a leading letter, as in case of the variable loop such as e11, e12 etc. (see, for example, Sprinzl M, Horn C, Brown M, Ioudovitch A, Steinberg S. Compilation of tRNA sequences and sequences of tRNA genes. Nucleic Acids Res. 1998;26(1): 148-53). In the following, the tRNA-specific numbering will also be referred to as "tRNA numbering convention" or "transfer RNA numbering convention".

The terms "synthetic transfer ribonucleic acid" or "synthetic tRNA" refer to an in-vitro synthesized tRNA and/or a non-naturally occurring tRNA. The term also encompasses analogues to naturally occurring tRNAs, i.e. tRNAs being structurally similar to naturally occurring tRNAs, but being modified in the base component, the sugar component and/or the phosphate component of one or more of the nucleotides, of which the tRNA is composed. The modified tRNA may, for example, have the phosphodiester backbone modified in that the phosphodiester bridge is replaced by a phosphorothioate, phosphoramidate or methyl phosphonate bridge. The sugar component may, for example, be modified at the 2' OH group, e.g. by dehydroxylating it to a deoxy ribonucleotide, or by replacing it with a methoxy-, methoxyethoxy- or aminoethoxy group. A synthetic transfer ribonucleic acid can, for example, be synthesized chemically and/or enzymatically in vitro, or in a cell based system, e.g. in a bacterial cell in vivo.

The term "modified nucleotide" (or "unusual nucleotide") in reference to tRNA relates to a nucleotide having modified or unusual nucleotide bases, i.e. other than the usual bases adenine (A), uracil (U), guanine (G) and cytosine (C), and/or a nucleotide having a chemically modified sugar (ribose or deoxyribose) moiety, for example, a methylated ribose component. Examples of modified nucleotides and some of the abbreviations used to denote these modified nucleotides are given in the following table 1:

**Table 1. Modified nucleotides and some of the abbreviations in use.**

| | |
|---|---|
| Archaeosine | G^{∗}, G+ |
| N4-acetylcytidine | ac4c |
| 5-(carboxyhydroxymethyl)uridine | chm5u |
| 2'-O-methylcytidine | cm, Cm, 2'-OMeC |
| 5 -carb oxy methy methylaminomethyl-2-thiouridine | cmnm5 s2u |
| 5-carboxymethylaminomethyluridine | cmnm5u |
| dihydrouridine | D, d |
| 2'-O-methylpseudouridine | fm, 2'-OMeΨ, Ψm |
| beta-D-galactosylqueuosine | gal q, galQ |
| 2'-O-methylguanosine | gm, Gm, 2'-OMeG |
| 2'-O-methyl-5-hydroxymethylcytidine | hm5cm |
| 5-hydroxymethylcytidine | hm5c |
| inosine | I, I |
| N6-isopentenyladenosine | i6a, i6A |
| 1-methyladenosine | m1a, m1A |
| 1-methylpseudouridine | m1f, m1Ψ |
| 1-methylguanosine | m1g, m1G |
| 1-methylinosine | mli |
| N2,N2-dimethylguanosine | m22g |
| 2'-O-methyladenosine | am, 2'-OMeA |
| N2-methyladenosine | m2a, m2A |
| N2-methylguanosine | m2g, m2G |
| 3-methylcytidine | m3c, m3C |
| 5-methylcytidine | m5c, m5C |
| 5-methyluridine | m5u, m5U |
| N6-methyladenosine | m6a, m6A |
| 7-methylguanosine | m7g, m7G |
| 5-methylaminomethyluridine | mam5u |
| 5-methoxyaminomethyl-2-thiouridine | mam5s2u |
| beta-D-mannosylqueuosine | man q, manQ |
| 5-methoxycarbonylmethyl-2-thiouridine | mcm5s2u |
| 5-methoxycarbonylmethyluridine | mcm5u |
| 5-methoxycarbonylmethyl-2'-O-methyluridine | mcm5um |
| 5-(carboxyhydroxymethyl)uridine methyl ester | mchm5u |
| 5-carbamoylmethyluridine | ncm5U |
| 5-carbamoylmethyl-2'-O-methyluridine | ncm5Um |
| 5-methoxyuridine | mo5u |
| 2-methylthio-N6-isopentenyladenosine | ms2i6a |
| 2-methylthio-N6-threonylcarbamoyladenosine | ms2t6a |
| N-((9-beta-D-ribofuranosylpurine-6-yl)N-methylcarbamoyl)threonine | mt6a |
| N6-methyl-N6-threonylcarbamoyl adenosine | m6t6a |
| uridine-5-oxyacetic acid-methylester | mv |
| uridine-5-oxyacetic acid | o5u |
| wybutoxosine | osyw |
| pseudouridine | p, Ψ |
| queuosine | q, Q |
| 2-thiocytidine | s2c |
| 5-methyl-2-thiouridine | s2t |
| 2-thiouridine | s2u |
| 4-thiouridine | s4u |
| 5-methyluridine | t |
| N6-Threonylcarbamoyladenosine | t6a, t6A |
| 2'-O-methyl-5-methyluridine | tm |
| 2'-O-methyluridine | um, 2'-OMeU |
| wybutosine | yw, Y |
| hydroxywybutosine | OHyw, OHyW |
| peroxywybutosine | o2yW, o2yw |
| 3-(3-amino-3-carboxy-propyl)uridine | acp3u, x |

Upper case letters may be used in abbreviations instead of lower case letters, e.g. Gm may synonymously be used for gm, m5C for m5c etc.

The terms "modifications" or "nucleotide modifications" used herein in relation to a tRNA of the invention relates to single modified nucleotides, combinations of two or more modified nucleotides not forming base-pairs, or to one or more pairs of modified nucleotides. The terms "pair of modified nucleotides" or "modified nucleotide pair" relate to two modified nucleotides forming a base-pair within the tRNA, when correctly folded.

The term "unique" in relation to a modified nucleotide or combination of nucleotides relates to the uniqueness of a certain modified nucleotide at a certain position in a tRNA or a certain combination of modified nucleotides at the positions concerned.

The term "corresponding modified nucleotide" relates to a modified nucleotide at a given position in a sequence, which base has been modified based on the usual, i.e. unmodified, base of the nucleotide at the same position in the original sequence to be compared with the sequence containing the modified nucleotide. A corresponding modified nucleotide is thus any nucleotide that, in a cell, is usually produced from a usual nucleotide by modifying the usual nucleotide. A modified nucleotide corresponding to uridine, for example, is thus any nucleotide derived by the modification of uridine. As an example, 5-(carboxyhydroxymethyl)uridine (chm5u) at a particular position in a sequence may be a modified nucleotide corresponding to uridine at the same position in the original sequence. Further modified nucleotides corresponding to uridine are, for example, 5-methyluridine (t), 2'-O-methyl-5-methyluridine (tm), 2'-O-methyluridine (um), or 5-methoxyuridine (mo5u). Inosine, as another example, is produced from adenosine and thus is a modified nucleotide corresponding to adenosine.

The term "codon" refers to a sequence of nucleotide triplets, i.e. three DNA or RNA nucleotides, corresponding to a specific amino acid or stop signal during protein synthesis. A list of codons (on mRNA level) and the encoded amino acids are given in the following:

| Amino acid | One Letter Code | Codons |
|---|---|---|
| Ala | A | GCU, GCC, GCA, GCG |
| Arg | R | CGU, CGC, CGA, CGG, AGA, AGG |
| Asn | N | AAU, AAC |
| Asp | D | GAU, GAC |
| Cys | C | UGU, UGC |
| Gln | Q | CAA, CAG |
| Glu | E | GAA, GAG |
| Gly | G | GGU, GGC, GGA, GGG |
| His | H | CAU, CAC |
| Ile | I | AUU, AUC, AUA |
| Leu | L | UUA, UUG, CUU, CUC, CUA, CUG |
| Lys | K | AAA, AAG |
| Met | M | AUG |
| Phe | F | UUU, UUC |
| Pro | P | CCU, CCC, CCA, CCG |
| Ser | S | UCU, UCC, UCA, UCG, AGU, AGC |
| Thr | T | ACU, ACC, ACA, ACG |
| Trp | W | UGG |
| Tyr | Y | UAU, UAC |
| Val | V | GUU, GUC, GUA, GUG |

| | | |
|---|---|---|
| START: AUG STOP: UAA, UGA, UAG, abbreviated "X" | | |

The term "sense codon" as used herein refers to a codon coding for an amino acid. The term "stop codon" or "nonsense codon" refers to a codon, i.e. a nucleotide triplet, of the genetic code not coding for one of the 20 amino acids normally found in proteins and signalling the termination of translation of a messenger RNA.

The term "frameshift mutation" refers to an out-of-frame insertion or deletion (collectively called "indels") of nucleotides with a number not evenly divisible by three. This perturbs the nucleotide sequence decoding which proceeds in steps of three nucleotide bases. The term "-1 frameshift mutation" relates to the deletion of a single nucleotide causing a shift in the reading frame by one nucleotide leading to the first nucleotide of the following codon being read as part of the codon from which the nucleotide has been deleted. Deletion of one nucleotide from the upstream codon along with several triplets (i.e. deletion of 4, 7, 10 etc. nucleotides) is also considered as -1 frameshifting. The term "+1 frameshift mutation" relates to the insertion of a single nucleotide into a triplet, or the deletion of two nucleotides. The result of either event is to shift the reading frame by one nucleotide, such that a nucleotide of an upstream codon is being read as part of a downstream codon. Insertion of one nucleotide along with several triplets (3*n*+ 1 nucleotides, n being an integer, i.e. insertion of 4, 7, 10 etc. nucleotides), or deletion of two nucleotides from the upstream codon along with several triplets (i.e. deletion of 5, 8, 11 etc nucleotides) is also considered as +1 frameshifting.

The term "anticodon" refers to a sequence of usually three nucleotides within a tRNA that base-pair (non-covalently bind) to the three bases (nucleotides) of the codon on the mRNA. In a natural tRNA a standard three-nucleotide anticodon is usually represented by the nucleotides at positions 34, 35 and 36. An anticodon may also contain nucleotides with modified bases. The terms "four nucleotide anticodon" or "four base anticodon" relate to an anticodon having four consecutive nucleotides (bases) which pair with four consecutive bases on an mRNA. The terms "quadruplet nucleotide anticodon" or "quadruplet anticodon" may also be used to denote a "four nucleotide anticodon". The terms "five nucleotide anticodon" or "five base anticodon" relate to an anticodon having five consecutive nucleotides (bases) which bind (base-pair) to five consecutive bases on an mRNA. The terms "quintuplet nucleotide anticodon" or "quintuplet anticodon" may also be used to denote a "five nucleotide anticodon".

The term "anticodon arm" refers to a part of the tRNA comprising the anticodon. The anticodon arm is composed of a stem portion ("anticodon stem"), usually consisting of five base pairs (positions 27-31 and 39-43), and a loop portion ("anticodon loop"), i.e. a consecutive sequence of unpaired nucleotides bound to the anticodon stem. The anticodon arm takes the positions 27 to 43, the position numbering following transfer RNA numbering convention.

The term "anticodon loop" refers to the unpaired nucleotides of the anticodon arm containing the anticodon. Naturally occurring tRNAs usually have seven nucleotides in their anticodon loop, three of which pair to the codon in the mRNA.

The term "extended anticodon loop" refers to an anticodon loop with a higher number of nucleotides in the loop than in naturally occurring tRNAs. An extended anticodon loop may, for example, contain more than seven nucleotides, e.g. eight, nine, or ten nucleotides. In particular, the term relates to an anticodon loop comprising an anticodon composed of more than three consecutive nucleotides, e.g. four, five or six nucleotides, being able to base-pair with a corresponding number of consecutive nucleotides in an mRNA.

The term "anticodon stem" refers to the paired nucleotides of the anticodon arm that carry the anticodon loop.

The term "T arm" relates to a portion of a tRNA composed of a stem portion ("T stem") and a loop portion ("T loop") between the acceptor stem and the variable loop. The T arm takes the positions 49 to 65, the position numbering following transfer RNA numbering convention. The T stem is usually composed of five nucleotide pairs (taking positions 49-53 and 61-65).

The term "T stem" (or "TψC" stem) relates to the paired nucleotides of the T arm, which carry the T loop, i.e. the unpaired nucleotides of the T arm.

The term "D arm" relates to the tRNA portion composed of a stem ("D stem"), i.e. the paired nucleotides of the D arm, and a D loop, i.e. the unpaired nucleotides of the D arm, between the acceptor stem and the anticodon arm. The D arm takes the positions 10-25, the position numbering following transfer RNA numbering convention.

The term "variable loop" refers to a tRNA loop located between the anticodon arm and the T arm. The number of nucleotides composing the variable loop may largely vary from tRNA to tRNA. The variable loop can thus be rather short or even missing, or rather large, forming, for example, a helix. The term "variable arm" may synonymously be used for the term "variable loop". Use of the term "variable loop" does not exclude the existence of a "stem" portion within the variable loop, i.e. a stretch of consecutive nucleotides of the variable loop forming base pairs with a complementary stretch of other consecutive nucleotides of the variable loop. The variable loop takes the positions 44 to 48, the position numbering following transfer RNA numbering convention. It should be noted that the variable loop may have a considerable number of additional nucleotides that are separately numbered (see Fig. 1).

The terms "codon base triplet" or "anticodon base triplet", when used herein, refer to sequences of three consecutive nucleotides which form a codon or anticodon. Synonymously, the terms "three nucleotide codon" (also "three base codon") or "three nucleotide anticodon" (also "three base anticodon"), or abbreviations thereof, e.g. "3nt codon" or "3nt anticodon", may be used.

The term "base pair" refers to a pair of bases, or the formation of such a pair of bases, joined by hydrogen bonds. One of the bases of the base pair is usually a purine, and the other base is usually a pyrimidine. In RNA the bases adenine and uracil can form a base pair and the bases guanine and cytosine can form a base pair. However, the formation of other base pairs ("wobble base pairs") is also possible, e.g. base pairs of guanine-uracil (G-U), hypoxanthine-uracil (I-U), hypoxanthine-adenine (I-A), and hypoxanthine-cytosine (I-C). The term "being able to base-pair" refers to the ability of nucleotides or sequences of nucleotides to form hydrogen-bond-stabilised structures with a corresponding nucleotide or nucleotide sequence.

The term "base", as used herein, for example in terms like "the bases A, C, G or U", encompasses or is synonymously used to the term "nucleotide", unless the context clearly indicates otherwise.

"PTC" refers to a premature termination codon. This is a stop codon introduced into a coding nucleic acid sequence by a nonsense mutation, i.e. a mutation in which a sense codon coding for one of the twenty proteinogenic amino acids specified by the standard genetic code is changed to a chain-terminating codon. The term thus refers to a premature stop signal in the translation of the genetic code contained in mRNA. The term "premature stop codon" ("PSC") may be used synonymously for a premature termination codon, PTC.

The terms "frameshift suppression" or "frameshift rescue" refer to mechanisms masking the effects of a frameshift mutation and at least partly restoring the wild-type phenotype.

The terms "nonsense suppression", "nonsense mutation suppression", "PTC suppression" or "PTC rescue" refer to mechanisms masking the effects of a nonsense mutation and at least partly restoring the wild-type phenotype.

The term "tRNA sequestration" relates to the irreversible binding of a tRNA to a tRNA synthetase such that the tRNA binds to the tRNA synthetase, but is not or essentially not released from the tRNA synthetase.

The term "suppressor tRNA" relates to a tRNA altering the reading of a messenger RNA in a given translation system such that, for example, a frameshift or nonsense mutation is "suppressed" and the effects of the frameshift or nonsense mutation are masked and the wild-type phenotype at least partly restored. An example of a suppressor tRNA is a tRNA carrying an amino acid and being able to base-pair to a PTC in an mRNA or, in case of a tRNA with an extended anticodon loop and a four-, five- or six-base anticodon, for example, to a section on an mRNA having two consecutive codons, one or both of which being mutated, or having a first and consecutive second codon, of which one is intact and one has an insertion or deletion. The translation system can thus correct the reading frame in the case of frameshift mutation or read through a PTC in the case of non-sense mutation.

The term "decoding activity" in relation to a tRNA refers to the property or capacity of a transfer RNA to be used by the translation machinery of a cell as an amino acid donor for the production of a protein. A tRNA has a "decoding activity" if, under physiologic conditions, it is charged with a cognate amino acid and the charged tRNA (aminoacyl-tRNA) is subsequently incorporated into a protein. The decoding activity of a first tRNA for a given amino acid can, for example, be compared with the decoding activity of a second tRNA for the same amino acid by comparing the proportion at which the amino acid of the first or second tRNA is incorporated in the protein. The terms "rescue activity" or "readthrough activity" are used herein in relation to the decoding activity of a suppressor tRNA, e.g. a frameshift suppressor or nonsense suppressor tRNA, in particular a tRNA designed to decode a premature stop codon in an mRNA into an amino acid, such that translation of the mRNA into the corresponding protein is not prematurely interrupted.

The term according to which the tRNA of the invention "still serves its function in the translational machinery of a living cell" relates to a tRNA having a decoding activity. In case of a suppressor tRNA, the term relates to a tRNA having a rescue activity greater than the spontaneous (stochastic) rescue activity in a given cellular environment.

The term "homology" in relation to a nucleic acid refers to the degree of similarity or identity between the nucleotide sequence of the nucleic acid and the nucleotide sequence of another nucleic acid. Homology is determined by comparing a position in the first sequence with a corresponding position in the second sequence in order to determine whether identical nucleotides are present at that position. It may be necessary to take sequence gaps into account in order to produce the best possible alignment. For determining the degree of similarity or identity between two nucleic acids it is preferable to take a minimum length of the nucleic acids to be compared into account, for example at least 25%, 30%, 40%, 50%, 60%, 70%, 80%, 90%, 91%, 92%, 93%, 94%, 95%, 96%, 97%, 98%, 99%, 99.2% or 99.5% of the nucleotides in the respective sequences. Preferably the full length of the respective nucleic acid(s) is used for comparison. The degree of similarity or identity of two sequences can be determined by using a computer program such as muscle (Edgar, R.C. (2004), Muscle: multiple sequence alignment with high accuracy and high throughput, Nucleic Acids Res., 32, 1792-1797, doi: 10.1093/nar/gkh340) or mafft (Katoh, K. and Standley, D.M. (2013) MAFFT Multiple Sequence Alignment Software Version 7, Mol. Biol. Evol., 30, 772-780, doi.org/10.1093/molbev/mst010). Where such terms like "x % homologous to" or "homology of x %" are used herein, this means that two nucleic acids have a sequence identity or similarity, preferably sequence identity, of x %, e.g. 50%.

The term "aminoacylation" relates to the enzymatic reaction in which a tRNA is charged with an amino acid. An aminoacyl tRNA synthetase (aaRS) catalyses the esterification of a specific cognate amino acid or its precursor to a compatible cognate tRNA to form an aminoacyl-tRNA. The term "aminoacyl-tRNA" thus relates to a tRNA with an amino acid attached to it. Each aminoacyl-tRNA synthetase is highly specific for a given amino acid, and, although more than one tRNA may be present for the same amino acid, there is only one aminoacyl-tRNA synthetase for each of the 20 proteinogenic amino acids. The terms "charge" or "load" may also be used synonymously for "aminoacylate". The term "aminoacylated" in relation to the synthetic tRNA of the invention relates to a synthetic tRNA already charged (precharged) with an amino acid or a dipeptide, such that the tRNA is already acylated when entering the target cell. The term "preaminoacylated" may synonymously be used in this context.

The synthetic tRNA of the invention comprises one or more modified nucleotides selected from features a) to o) above. This includes any combination of the modified nucleotides and

positions mentioned, for example any combination of any of the possible modified nucleotides at a first position given in the following table 2 with any of the possible modified nucleotides at one or more other positions given in the following table 2 (position numbering follows transfer RNA numbering convention):

**Table 2. List of possible modified nucleotides at different positions within the synthetic tRNA according to features a) to g) above. Position numbering follows transfer RNA numbering convention.**

| No. | Position | modified nucleotide |
|---|---|---|
| 1 | 11 | m5C |
| 2 | 11 | 2'-OMeC |
| 3 | 21 | m6A |
| 4 | 21 | m1A |
| 5 | 21 | 2'-OMeA |
| 6 | 56 | m5C |
| 7 | 56 | 2'-OMeC |
| 8 | 59 | m6A |
| 9 | 59 | m1A |
| 10 | 59 | 2'-OMeA |
| 11 | 60 | Ψ |
| 12 | 60 | 2'-OMeΨ |
| 13 | 60 | m5U |
| 14 | 60 | m1Ψ |
| 15 | 60 | 2'-OMeU |
| 16 | 73 | m5C |
| 17 | 73 | m6A |
| 18 | 73 | m5U |
| 19 | 73 | m1A |
| 20 | 73 | 2'-OMeA |
| 21 | 73 | 2'-OMeC |
| 22 | 73 | 2'-OMeU |
| 23 | 73 | 2'-OMeΨ |
| 24 | 73 | Ψ |
| 25 | 73 | m1Ψ |

A synthetic transfer RNA of the invention may thus include a single modified nucleotide according to table 1, for example Ψ at position 60 (No. 11 of table 2 above), or a combination of two, three, four or more modified nucleotides at the given positions, for example m5C or 2'-OMeC at position 11, and m6A, m1A or 2'-OMeA at position 21. A preferred embodiment of the synthetic transfer RNA of the invention has one of the modified nucleotides at position 60 mentioned in table 2, i.e. Ψ, 2'-OMeΨ, m5U, m1Ψ or 2'-OMeU, particular preferred Ψ. Further preferred, alternatively or in addition to any of the above-mentioned modified nucleotides at position 60 or any of the modified nucleotides at the other positions mentioned in tables 2-4, the synthetic transfer RNA of the invention has one of the modified nucleotides Ψ, 2'-OMeΨ, m5U, m1Ψ, or 2'-OMeU at position 37. This is particular preferred in embodiments of a synthetic tRNA of the invention which are configured to be used as suppressor tRNA, e.g. a PTC suppressor tRNA.

Combinations of modified nucleotides according to features h) to o) relate to positions of nucleotides forming a base pair within the synthetic tRNA, i.e. nucleotides bonded together with hydrogen bonds, e.g. in the acceptor stem (positions 2, 71; 5, 68), the anticodon stem (positions 29, 41) or the T stem (positions 49, 65). The combinations are given in the tables below.

**Table 3. List of possible modified nucleotide pairs and their positions within the synthetic tRNA.**

| | Positions | | | |
|---|---|---|---|---|
| No. | 1^{St} | 2^{nd} | mod. nucl. 1^{st} pos. | mod. nucl. 2^{nd} pos. |
| 1 | 49 | 65 | m6A, m1A, 2'-OMeA | Ψ, 2'-OMeΨ, m5U, m1Ψ, or 2'-OMeU |
| 2 | 65 | 49 | m6A, m1A, 2'-OMeA | Ψ, 2'-OMeΨ, m5U, m1Ψ, 2'-OMeU |
| 3 | 2 | 71 | Ψ, 2'-OMeΨ, m5U, m1Ψ 2'-OMeU | m6A, m1A, 2'-OMeA |
| 4 | 71 | 2 | Ψ, 2'-OMeΨ, m5U, m1Ψ, 2'-OMeU | m6A, m1A, 2'-OMeA |
| 5 | 5 | 68 | Ψ, 2'-OMeΨ, m5U, m1Ψ, 2'-OMeU | m6A, m1A or 2'-OMeA |
| 6 | 68 | 5 | Ψ, 2'-OMeΨ, m5U, m1Ψ, 2'-OMeU | m6A, m1A or 2'-OMeA |
| 7 | 29 | 41 | m6A, m1A, 2'-OMeA | Ψ, 2'-OMeΨ, m5U, m1Ψ, or 2'-OMeU |
| 8 | 41 | 29 | m6A, m1A, 2'-OMeA | Ψ, 2'-OMeΨ, m5U, m1Ψ, or 2'-OMeU |

It should be noted that also any possible combination of the pairs given in table 3 are encompassed, e.g. a combination of No. 1 and 3, No. 2 and 3, and No. 2 and 4, No. 6 and 7, No. 1, 3 and 6 of table 3 etc. Further, it should be noted that the modified nucleotides can independently be selected from the possible modified nucleotides mentioned in table 3. As an example, m6A can be chosen for position 49 and Ψ for position 65. Alternatively, for example, m6A could be chosen for position 49 and m5U for position 65. Still further, it should be noted for clarity that any of the pairs or combination of pairs of modified nucleotides mentioned in table 3 can, of course, be combined with any of the modifications and any combination of modifications listed in table 2.

The following table 4 lists possible individual combinations of pairs of modified nucleotides according to table 3 above. The terms "1^{st} position" and "2^{nd} position" relate to the positions of table 3 above.

**Table 4: Possible pairwise combinations of modified nucleotides. 1^{st} position and 2^{nd} position stand for the positions given in table 3 above.**

| No. | mod. nucl. 1^{st} pos. | mod. nucl. 2^{nd} pos |
|---|---|---|
| 1 | 2'-OMeA | Ψ |
| 2 | 2'-OMeA | 2'-OMeΨ |
| 3 | 2'-OMeA | m5U |
| 4 | 2'-OMeA | m1Ψ |
| 5 | 2'-OMeA | 2'-OMeU |
| 6 | m1A | Ψ |
| 7 | m1A | 2'-OMeΨ |
| 8 | m1A | m5U |
| 9 | m1A | m1Ψ |
| 10 | m1A | 2'-OMeU |
| 11 | m6A | Ψ |
| 12 | m6A | 2'-OMeΨ |
| 13 | m6A | m5U |
| 14 | m6A | m1Ψ |
| 15 | m6A | 2'-OMeU |
| 16 | 2'-OMeU | 2'-OMeA |
| 17 | 2'-OMeU | m1A |
| 18 | 2'-OMeU | m6A |
| 19 | 2'-OMeΨ | 2'-OMeA |
| 20 | 2'-OMeΨ | m1A |
| 21 | 2'-OMeΨ | m6A |
| 22 | m1Ψ | 2'-OMeA |
| 23 | m1Ψ | m1A |
| 24 | m1Ψ | m6A |
| 25 | m5U | 2'-OMeA |
| 26 | m5U | m1A |
| 27 | m5U | m6A |
| 28 | Ψ | 2'-OMeA |
| 29 | Ψ | m1A |
| 30 | Ψ | m6A |

The synthetic transfer RNA of the invention has a least a single nucleotide modification or a combination of nucleotide modifications, selected from the list of nucleotide modifications presented in features a) to o) above or from tables 2 to 4 above, that is not present in known tRNAs, at least not in naturally occurring human tRNAs. It is, however, preferred that the synthetic transfer RNA otherwise has a pattern of nucleotide modifications characteristic for naturally occurring tRNA of a target species, in particular human tRNA. This is useful in order to avoid an unwanted immune response in a given organism, e.g. a human organism, and to minimize adverse effects on decoding activity.

As mentioned above, it is preferred that the synthetic tRNA of the invention comprises, in addition to the unique modifications described herein, further modified nucleotides that are known to be present in naturally occurring tRNAs, preferably in human tRNAs, at known positions. Although it is preferred that the known modifications are introduced into the synthetic tRNA of the invention in vitro, it is also possible to only introduce at least one of the unique modifications described herein chemically or enzymatically in vitro and to subsequently let the tRNA be further modified within a living cell. The latter also encompasses that the further modifications are introduced in vitro by isolated cells into which the tRNA has been biotechnologically introduced, or that the tRNA is further modified in vivo within the cells of a living body, e.g. a human body, into which the tRNA has been introduced. In case of a further modification of a synthetic tRNA by cells in vitro the tRNA may be isolated from the cells after modification has taken place.

A list of known modified nucleotides in human tRNAs and their positions is given in Table 4 below.

**Table 5. Examples of known modified nucleotides and positions within cytoplasmic human tRNA (position numbering according to the specific tRNA numbering convention for a generalized "consensus" tRNA, see also Fig. 1; see, for example, Suzuki, T. The expanding world of tRNA modifications and their disease relevance. Nat Rev Mol Cell Biol 22, 375-392 (2021). https://doi.org/10.1038/s41580-021-00342-0).**

| Position | Modification |
|---|---|
| 4 | um, am |
| 6 | m2g |
| 7 | m2g |
| 9 | m1a, m1g |
| 10 | m2g |
| 12 | ac4c |
| 13 | Ψ |
| 14 | m1a |
| 16 | d |
| 17 | d |
| 18 | gm |
| 20 | d,acp3u |
| 20a | d,acp3u |
| 20b | Ψ |
| 26 | m22g, m2g |
| 27 | Ψ, m22g, m5c |
| 28 | Ψ |
| 30 | Ψ |
| 31 | Ψ |
| 32 | Ψ, Ψm, cm, um, m3c |
| 34 | I, Ψ, m5c, cm, gm, 2'O-methylribose, q, mcm5u, ncm5u, mcm5um, mcm5s2u, mchm5u, manQ, galQ, hm5cm, hm5c |
| 35 | Ψ |
| 37 | i6A, t6A, m1g, mli, OHyW, o2yW, m6t6A, ms2t6A |
| 38 | Ψ, m5c |
| 39 | um, gm, Ψ, Ψm |
| 44 | um |
| 46 | m7g |
| 47 | d |
| 48 | m5c |
| 49 | m5c |
| 50 | m5c, Ψ |
| 54 | m5u, m5um, Ψ |
| 55 | Ψ |
| 58 | m1a |
| 72 | Ψ, m5c |

It is preferred that a total of 1 to 25%, further preferred 1-20%, 1-15% or 1-10%, of the nucleotides that make up the synthetic tRNA of the invention, including the at least one modified nucleotide or pair of modified nucleotides according to features a) to o) above are modified nucleotides.

It is preferred that the synthetic tRNA of the invention contains, in addition to the unique modified nucleotides or nucleotide combinations mentioned in tables 2 to 4, known modified nucleotides at known positions, for example from the list of table 5 above, in order to have a nucleotide modification pattern that comes as close as possible to a known nucleotide modification pattern of a tRNA of a target species, in particular human tRNA.

For example, it is preferred that the tRNA of the invention includes dihydrouridine (d) at position 16, and/or dihydrouridine (d) at position 17, and/or 2'-O-methylguanosine (Gm, 2'-OMeG) at position 18, and/or dihydrouridine (d) at position 20 and/or 20a, and/or a pseudouridine (Ψ) at position 28, and/or a pseudouridine (Ψ) at position 30, and/or a pseudouridine (Ψ) at position 31, and/or a pseudouridine (Ψ) or 2'-O-methylpseudouridine (Ψm) at position 39, and/or 5-methylcytidine (m5c) at position 48, and/or a 2'-O-methyluridine (Um, 2'-OMeU) at position 44, and/or pseudouridine (Ψ) or 5-methyluridine (m5u) at position 54, and/or a pseudouridine (Ψ) at position 55. The synthetic tRNA of the invention may contain any combination of these modified nucleotides at the given positions, for example, two or three of them. Further preferred, the tRNA of the invention includes all these modified nucleotides at the given positions.

Further preferred, the synthetic tRNA of the invention includes at least one m5c nucleotide, be it at any of the unique positions 11, 56 or 73 (see table 2) or at any other position in the tRNA, preferably at a position where m5c is known to occur in natural human tRNA, e.g. at position 27, 34, 38, 48, 49, or 72 (see table5). Further preferred, the synthetic tRNA of the invention has m5c at position 72, m5C at position 38, m5C at position 48, m5C at position 49, or m5C at position 50, or a combination of any number of these modified nucleotides at these positions. It is further preferred that the synthetic tRNA of the invention includes, alternatively or additionally, at least one of the modified nucleotides m1A, m6A, Ψm or pseudouridine (Ψ) at one of the positions given in tables 2-5 for those modified nucleotides. Still further preferred, the synthetic tRNA of the invention does not include Cm at position 56 and/or m1Ψ at position 54, and/or archaeosine (G^{∗}) at position 15. Particularly preferred, the synthetic tRNA of the invention includes at least one m5c nucleotide or combination of m5c nucleotides as described above, and does not include Cm at position 56 and/or m1Ψ at position 54. Further preferred, the synthetic tRNA of the invention additionally includes at least one of the modified nucleotides m1A, m6A, Ψm or pseudouridine (Ψ) at one of the positions given in tables 2-5 for those modified nucleotides, as mentioned above.

The synthetic transfer RNA of the invention may be synthesized in vitro chemically and/or enzymatically, for example by splinted ligation (see, for example, Kershaw CJ, O'Keefe RT.

Splint ligation of RNA with T4 DNA ligase. Methods Mol Biol. 2012;941:257-269. doi:10.1007/978-1-62703-113-4_19). The synthetic tRNA of the invention is designed, for example computationally, in such a manner that an aminoacyl tRNA synthetase that naturally occurs in a living cell, preferably a mammalian cell, e.g. a human cell, is able to charge the tRNA with a specific desired amino acid.

The synthetic transfer RNA of the invention may have a "normal" anticodon arm, i.e. an anticodon arm including 10 nucleotides forming the anticodon stem and 7 nucleotides forming the anticodon loop containing a three-base anticodon base-pairing with a respective codon on an mRNA. The anticodon can, for example, be designed to base-pair to a premature stop codon on an mRNA, and function as a suppressor tRNA. Alternatively, the synthetic transfer RNA of the invention can, however, also have an extended anticodon loop having a four-base, five-base or six-base anticodon (see WO 2019/175316 A1 and WO 2020/208169 A1, the disclosures of which are incorporated by reference herein in their entirety).

In preferred embodiments, the synthetic tRNA of the invention may be structurally modified in that the tRNA has a structurally modified D arm, anticodon arm, variable loop and/or T arm. "Structurally modified" means that the tRNA of the invention contains an anticodon arm, and/or a variable loop and/or a T-arm and/or a D arm that has been modified, i.e. has a nucleotide sequence, for example of the stem portion or the loop portion, that differs from the D arm and/or the anticodon arm and/or the variable loop and/or the T arm of a comparable naturally occurring tRNA. "Comparable" tRNAs are tRNAs being aminoacylated by the same aminoacyl-tRNA synthetases. Particular preferred, the tRNA of the invention contains a D arm, an anticodon arm and/or a variable loop and/or a T arm that does not occur in natural tRNAs.

The anticodon arm, for example may have one of the following general structures with a modified stem portion:
5'-GCAGG-AC-Loop-CCUGU
5'-UUGGG-AC-Loop-CUCAA
5'-UUGGA-AC-Loop-UUCAA
5 '-AUGGU-AC-Loop-ACCAU

Preferred anticodon arms may have the following general sequences:

| | |
|---|---|
| GCAGGNNNNNNNCCUGU | (SEQ ID NO: 01) |
| GCAGGNNNNNNNNCCUGU | (SEQ ID NO: 02) |
| GCAGGNNNNNNNNNCCUGU | (SEQ ID NO: 03) |
| GCAGGNNNNNNNNNNCCUGU | (SEQ ID NO: 04) |
| UUGGGNNNNNNNCUCAA | (SEQ ID NO: 05) |
| UUGGGNNNNNNNNCUCAA | (SEQ ID NO: 06) |
| UUGGGNNNNNNNNNCUCAA | (SEQ ID NO: 07) |
| UUGGGNNNNNNNNNNCUCAA | (SEQ ID NO: 08) |
| UUGGANNNNNNNUUCAA | (SEQ ID NO: 09) |
| UUGGANNNNNNNNUUCAA | (SEQ ID NO: 10) |
| UUGGANNNNNNNNNUUCAA | (SEQ ID NO: 11) |
| UUGGANNNNNNNNNNUUCAA | (SEQ ID NO: 12) |
| AUGGUNNNNNNNACCAU | (SEQ ID NO: 13) |
| AUGGUNNNNNNNNACCAU | (SEQ ID NO: 14) |
| AUGGUNNNNNNNNNACCAU | (SEQ ID NO: 15) |
| AUGGUNNNNNNNNNNACCAU | (SEQ ID NO: 16) |

Underlined N's represent a 3nt, 4nt, 5nt or 6nt anticodon. N = A, C, G or U, or any modified base.

In preferred embodiments, the synthetic tRNA of the invention comprises, for example, an anticodon (AC) arm of one of the following sequences:

| | |
|---|---|
| GCAGGCUNNNAACCUGU | (SEQ ID NO: 17) |
| GCAGGCUNNNNAACCUGU | (SEQ ID NO: 18) |
| GCAGGCUNNNNNAACCUGU | (SEQ ID NO: 19) |
| GCAGGCUNNNNNNAACCUGU | (SEQ ID NO: 20) |
| UUGGGCUNNNAACUCAA | (SEQ ID NO: 21) |
| UUGGGCUNNNNAACUCAA | (SEQ ID NO: 22) |
| UUGGGCUNNNNNAACUCAA | (SEQ ID NO: 23) |
| UUGGGCUNNNNNNAACUCAA | (SEQ ID NO: 24) |
| UUGGACUNNNAAUUCAA | (SEQ ID NO: 25) |
| UUGGACUNNNNAAUUCAA | (SEQ ID NO: 26) |
| UUGGACUNNNNNAAUUCAA | (SEQ ID NO: 27) |
| UUGGACUNNNNNNAAUUCAA | (SEQ ID NO: 28) |
| AUGGUCUNNNAAACCAU | (SEQ ID NO: 29) |
| AUGGUCUNNNNAAACCAU | (SEQ ID NO: 30) |
| AUGGUCUNNNNNAAACCAU | (SEQ ID NO: 31) |
| AUGGUCUNNNNNNAAACCAU | (SEQ ID NO: 32) |

The underlined N's represent a 3nt, 4nt, 5nt or 6nt anticodon. N = A, C, G or U, or any modified base. A preferred 3nt anticodon is, for example, UCA for a tRNA acting as a PTC suppressor tRNA.

It is to be noted that the nucleotides may be modified such that the sequence contains corresponding modified nucleotides, for example at position 34 or 37 in the sequences above (position according to the tRNA numbering convention). Possible modifications are those listed in tables 2-5.

In a further preferred embodiment of the invention, the synthetic tRNA of the invention comprises, in combination with the AC arm mentioned above or alone, i.e. not in combination with the AC arm mentioned above, a variable loop (V loop) having the following sequence:
UGGGGUUUCCCCGC (SEQ ID NO: 33)
AGGGGAAACCCCGC (SEQ ID NO:34)

Again, the nucleotides may be modified such that the sequence contains corresponding modified nucleotides instead of the standard nucleotides given above.

It is particularly preferred that the tRNA of the invention does not include a V loop having any unmodified uridine nucleotide, i.e. any unmodified U.

In a further preferred embodiment of the invention, the synthetic tRNA of the invention comprises, in combination with one of the AC arm(s) described above and/or in combination with the V loop described above, or alone, i.e. not in combination with the AC arm(s) mentioned above and/or not in combination with the V loop mentioned above, a T arm having the following general sequence:
GCGGG-T-Loop-CCCGU
ACGGG-T-Loop-CCCGU

In preferred embodiments, the synthetic tRNA of the invention comprises, for example, a T arm of one of the following sequences

| | |
|---|---|
| GCGGG*UUCGAAU*CCCGU | (SEQ ID NO: 35) |
| ACGGG*UUCGAAU*CCCGU | (SEQ ID NO: 36) |
| *GCGGGUUCGAGU*CCCGU | (SEQ ID NO: 37) |
| *ACGGGUUCGAGU*CCCGU | (SEQ ID NO: 38) |

The loop part is in italics. The T arm has a stem of five base pairs. The nucleotides may be modified such that the sequence contains corresponding modified nucleotides instead of the standard nucleotides given above.

As mentioned above, for a given synthetic tRNA of the invention, the anticodon arm or at least the anticodon stem, the V loop and T arm, or at least the T stem, can be independently selected from the anticodon arms/stems, V loops and T arms/stems mentioned above. The synthetic tRNA of the invention may thus only include one of the anticodon arms/stems, the V loop or one of the T arms/stems mentioned above, for example only an anticodon arm with the sequence of SEQ ID NO: 17, or any combination thereof, for example an anticodon arm with the sequence of SEQ ID NO: 21 and a T arm having the sequence of SEQ ID NO:36.

It is to be noted here that the structurally modified anticodon arms, v-loop and T arms described above may or may not have any of the new modifications or modification combinations listed in tables 2-4 falling into these regions, although it is preferred that they have at least one of those modifications or a combination of those modifications. The structurally modified anticodon arms, v-loop and/or T arms can also be included in any synthetic tRNA independently of the presence of any of the modified nucleotides of tables 2-4, however, preferably with the proviso that the resulting tRNA differs otherwise in its sequence from any naturally occurring tRNA including any of the above anticodon arms, v-loop and /or T arms.

The skilled person is aware of the fact that a tRNA is aminoacylated with a specific amino acid by a specific aminoacyl tRNA synthetase (aaRS), and that the aaRS is able to recognize its cognate tRNA through unique identity elements at the acceptor stem and/or anticodon loop of the tRNA. In order to provide a tRNA which is loaded with its cognate amino acid in vivo, the skilled person will design the synthetic tRNA of the invention with suitable unique identity elements. It is also possible to aminoacylate a synthetic tRNA of the invention with a flexizyme and transfect it into an eukaryotic cell as aminoacyl-tRNA (Goto, Y., Kato, T. and Suga, H., Flexizymes for genetic code reprogramming, Nat Protoc., 6(6), 779, 2011, doi:10.1038/nprot.2011.331).

In the synthetic transfer RNA according to the invention the anticodon loop may be extended by a large enough number of nucleotides to accommodate a four-, five- or six-base anticodon. The anticodon loop of a synthetic transfer RNA of the invention may, for example, consist of 7-12, preferably 7 to 10 or 8-10, further especially preferred 8 or 9 nucleotides.

The synthetic transfer RNA according to the invention may be aminoacylated, i.e. carrying an amino acid or a dipeptide at the end of its acceptor stem. Preferably, the tRNA is aminoacylated with an amino acid being encoded by a sense codon base-pairing with the three-, four-, five- or six-base nucleotide anticodon of the synthetic tRNA, or with an desired amino acid or dipeptide, depending on the intended application. The synthetic tRNA of the invention can be chemically and/or enzymatically aminoacylated with a single amino acid or dipeptide. The loading of a tRNA with a dipeptide can be accomplished with methods known to those skilled in the art (see, for example, Maini R, Dedkova LM, Paul R, Madathil MM, Chowdhury SR, Chen S, Hecht SM, 2015, Ribosome-Mediated Incorporation of Dipeptides and Dipeptide Analogues into Proteins in Vitro, J. Am. Chem. Soc., 137, 11206-11209, doi 10.1021/jacs.5b03135). Engineered bacterial tRNA synthetases or RNA-based catalysts may, for example, be used to aminoacylate the tRNA with a dipeptide.

In a further aspect the invention relates to the synthetic transfer RNA according to the first aspect of the invention for use as a medicament. The transfer RNA of the invention is especially useful for treating patients with a disease associated with a nonsense mutation, i.e. a premature termination codon (PTC), or a frameshifting causing the absence of a functional protein or the dysfunction of a protein. Examples for diseases, in which the tRNA of the invention may advantageously be employed are Hurler syndrome (MPS I, ICD code E76.0, beta-thalassemia (ICD-10 code D56.1), neurofibromatosis type 1 (NF1, ICD-10 code Q85.0), Duchenne muscular dystrophy (DMD, ICD-10 code G71.0, Crohn's disease (CD, ICD-10 code K50), cystic fibrosis (CF, ICD-10 code E84), neuronal ceroid lipofuscinosis (NCL, ICD-10 code E75.4) and Tay-Sachs disease (TSD, ICD-10 code E75.0. The transfer RNA of the invention is also useful for treating patients with a disease which is at least partly caused by the sequestration of a tRNA leading to depletion of the cellular pool of the tRNA, e.g. Charcot-Marie-Tooth disease (CMT disease, ICD-10 code DG600).

Suitable compositions or means for delivering tRNAs to a cell are known. These include viral vectors such as adeno-associated virus (AAV)-based viral vectors, encapsulation in or coupling to nanoparticles, e.g. lipid nanoparticles.

In a further aspect the invention relates to a method of treating a person having a disease associated with a nonsense (PTC) or frameshift mutation, comprising administering an effective amount of the synthetic transfer RNA of the invention or of a composition comprising the synthetic transfer RNA of the invention to the person. In a preferred embodiment the method is for treating Hurler syndrome (MPS I, ICD code E76.0), beta-thalassemia (ICD-10 code D56.1), neurofibromatosis type 1 (NFl, ICD-10 code Q85.0), Duchenne muscular dystrophy (DMD, ICD-10 code G71.0), Crohn's disease (CD, ICD-10 code K50), cystic fibrosis (CF, ICD-10 code E84), neuronal ceroid lipofuscinosis (NCL, ICD-10 code E75.4) or Tay-Sachs disease (TSD, ICD-10 code E75.0).

The tRNA of the invention may, for example, also advantageously be used for treating a disease, e.g. hereditary motor and sensory neuropathy (Charcot-Marie-Tooth (CMT) disease), in which the natural tRNA is sequestered due to, for example, mutations in aminoacyl-tRNA-synthetase, and thus not or not sufficiently available for the translation machinery of the cell. The tRNA of the invention may, for example, be overexpressed in order to serve as a supplement for the sequestered tRNA (see, for example, Zuko A, Mallik M, Thompson R, Spaulding EL, Wienand AR, Been M, Tadenev ALD, van Bakel N, Sijlmans C, Santos LA, Bussmann J, Catinozzi M, Das S, Kulshrestha D, Burgess RW, Ignatova Z, Storkebaum E., 2021, tRNA overexpression rescues peripheral neuropathy caused by mutations in tRNA synthetase, Science 373(6559):1161-1166. Doi: 10.1126/science.abb3356).

In a still further aspect the invention relates to a synthetic transfer RNA comprising:
a) an anticodon arm having or comprising one of the sequences of SEQ ID NO: 01-16 or one of the sequences of SEQ ID NO: 17-32, and/or
b) a variable loop having or comprising one of the sequences of SEQ ID NO: 33-34, and/or
c) a T arm having or comprising one of the sequences according to SEQ ID NO: 35-38.

In a still further aspect the invention relates to a pharmaceutical composition, comprising a synthetic transfer RNA according to the first aspect of the invention, and a pharmaceutically acceptable carrier, for example lipid nanoparticles.

The invention will be described in the following by way of examples and the appended figures for illustrative purposes only.

Figure 1. Schematic drawing of a generalized "consensus" tRNA structure and its numbering according to tRNA numbering convention.

Figure 2. PTC-rescue of position-specific modified nonsense suppressor tRNAs as compared to the unmodified, in vitro transcribed tRNA for R208X UGA in Hep3B cells and S466X UGA in CFBE41o- cells.

Figure 3. IFN-a response in hPBMCs upon transfection with modified tRNAs. Mock transfection and a tRNA with uridine completely replaced by pseudouridine (PseudoU) served as negative control. Unmodified, in vitro transcribed tRNA served as a positive control. tRNAs were modified either at single-positions or stochastically across the whole sequence. Data are means ± SD (n=2).

Figure 1 depicts an example of a tRNA numbered according to the conventional numbering applied to a generalized "consensus" tRNA, beginning with 1 at the 5' end and ending with 76 at the 3' end. The tRNA is composed of tRNA nucleotides 11 and has the common cloverleaf structure of tRNA comprising an acceptor stem 2 with the CCA tail 10, a T arm 3 with the TψC loop 6, a D arm 4 with the D loop 7 and an anticodon arm 5 with a five-nucleotide stem portion 8 and the anticodon loop 9. In such a "consensus" tRNA the nucleotides of the natural anticodon triplet 25 are always at positions 34, 35 and 36, regardless of the actual number of previous nucleotides. A tRNA may, for example, contain additional nucleotides between positions 1 and 34, e.g. in the D loop, and in the variable loop 24 between positions 45 and 46.

Additional nucleotides may be numbered with added alphabetic characters, e.g. 20a, 20b etc. In the variable loop 24 the additional nucleotides are numbered with a preceding "e" and a following numeral depending on the position of the nucleotide in the loop. Positions of modified nucleotides within a tRNA of the invention are marked as black filled circles.

### Example 1

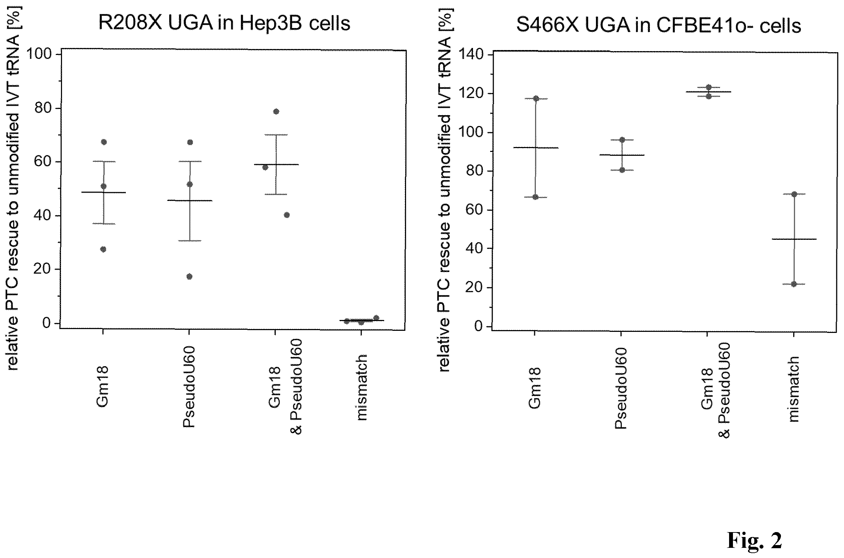

3 different nonsense suppressor tRNAs were synthesized containing either a single modification, 2'-O-Methylguanosine at position 18 (Gm18) or Pseudouridine (Ψ) at position 60 (PseudoU60), or a combination of both (Gm18 & PseudoU60). Through insertion of modifications the activity of the nonsense suppressor tRNAs should not be perturbed, thus, the PTC rescue activity was determined and compared to the unmodified, in vitro transcribed nonsense suppressor tRNA for which high PTC rescue activity had previously been already verified. PTC rescue of the tRNAs that were specifically modified at selected positions were reduced to ~50% for R208X in Hep3B cells, and to ~90% for S466X UGA in CFBE41o- cells (although here the mismatch control, that should not bind to the PTC and thus not rescue, is also higher). Interestingly, in both cases the combination of Gm18 together with PsuedoU60 showed higher PTC rescue as compared to the single modified tRNAs (Figure 2).

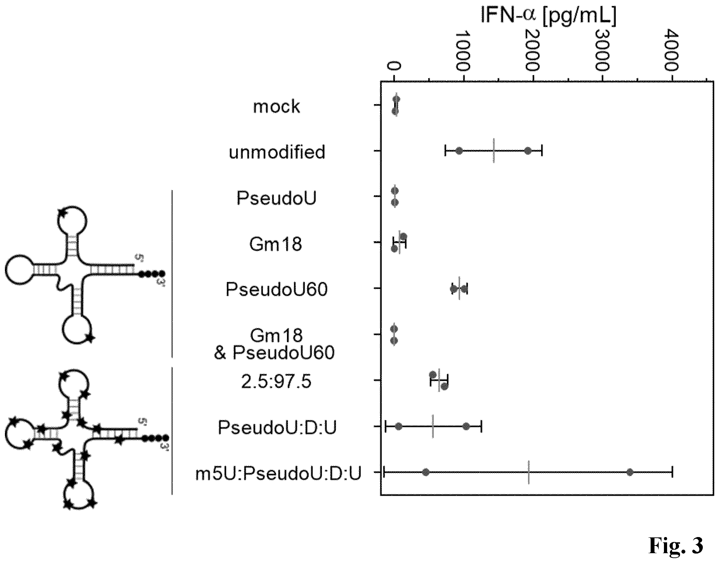

The immunostimulatory potential of different modified nonsense suppressor tRNAs was investigated in comparison to unmodified, in vitro transcribed tRNA (positive control). Mock transfection and a tRNA with uridine completely replaced by pseudouridine (PseudoU) served as negative control. As expected, unmodified tRNAs elicited an immune response, indicated by increased levels of IFN-a (Figure 3), a characteristic marker of the activated immune response. IFN-α levels of stochastically modified tRNAs (right) were increased as well as. However, two position-specific modified tRNAs showed IFN-α levels comparable to mock transfection or PseudoU-tRNA. This suggests that the incorporation of 2'-O-Methylguanosine at position 18 (Gm18) or a combination of Gm18 and PseudoU60 in tRNAs can be used to evade the immune response. It is to be noted that the IFN-a levels for the double combination of Gm18 and PseudoU60 are lower as compared to Gm18 alone, suggesting a synergistic effect of Gm18 and PseudoU60. Pseudo60 alone, however, stimulated the IFN-a response.

The immune-evasive effect of 2'-O-Methylguanosine at position 18 (Gm18), best followed by a G19, in tRNAs is already known (Jöckel, S. et al. The 2'-O-methylation status of a single guanosine controls transfer RNA-mediated toll-like receptor 7 activation or inhibition. J. Exp. Med. 209, 235-241 (2012); Rimbach, K., Kaiser, S., Helm, M., Dalpke, A. H. & Eigenbrod, T. 2'-O-Methylation within Bacterial RNA Acts as Suppressor of TLR7/TLR8 Activation in Human Innate Immune Cells. J. Innate Immun. 7, 482-493 (2015); Gehrig, S. et al. Identification of modifications in microbial, native tRNA that suppress immunostimulatory activity. J. Exp. Med. 209, 225-233 (2012); Kaiser, S., Rimbach, K., Eigenbrod, T., Dalpke, A. H. & Helm, M. A modified dinucleotide motif specifies tRNA recognition by TLR7. RNA 20, 1351-1355 (2014)). However, there is presently no evidence that a combination of Gm18 with PseudoU60 is immune-evasive. Thus, the presented results are based on a known tRNA modification (Gm18), but in combination with a modification at a novel position (PseudoU60), which is, to the knowledge of the inventors, never modified at least in humans, (Modomics database of RNA modifications, see Pietro Boccaletto, Magdalena A. Machnicka, Elzbieta Purta, Pawel Piatkowski, Blazej Baginski, Tomasz K. Wirecki, Valérie de Crécy-Lagard, Robert Ross, Patrick A. Limbach, Annika Kotter, Mark Helm, MODOMICS: a database of RNA modification pathways. 2017 update, Nucleic Acids Research 2017, 02 November, gkx1030, https://doi.org/10.1093/nar/gkx1030). The combination of both modifications showed higher PTC rescue and lower IFN-a levels as compared to the single modified tRNAs.

### tRNA synthesis with position-specific modifications

tRNAs with position-specific modification(s) were synthesized by splinted ligation of modified and unmodified tRNA fragments. Modified fragments were synthesized (Biomers). Unmodified fragments were produced by in vitro T7 transcription as described in Albers et al. 2021 (Albers, S. et al. Repurposing tRNAs for nonsense suppression, Nat. Commun. 12, 3850 (2021)).

Splinted ligation was performed as described in Kaiser et al. 2014 (Kaiser, S., Rimbach, K., Eigenbrod, T., Dalpke, A. H. & Helm, M. A modified dinucleotide motif specifies tRNA recognition by TLR7. RNA 20, 1351-1355 (2014)). Briefly, 3'-fragments were phosphorylated by incubation in KL buffer (50 mM Tris-HCl pH 7.4, 10 mM MgCl₂) supplemented with 5 mM ATP, 5 mM DTT and 0.75 units/µL T4 PNK (New England Biolabs) in a final volume of 150 µL for 1 hour. An equimolar amount of the 5'-fragment and the full-length cDNA splint were added in KL buffer supplemented with 5 mM ATP, 5 mM DTT to a volume of 500 µL. RNA fragments were hybridized to the cDNA splint by heating 75°C for 4 min and incubation at room temperature for 15 min. 1.5 U/µL T4 DNA ligase (New England Biolabs) and 0.3 U/µL T4 RNA ligase (New England Biolabs) were added and the ligation was performed at 16°C overnight. DNA was removed by addition of 1.5 U/µL DNase I (Thermo Fisher Scientific) and incubation at 37°C for 1 h. Full-length tRNAs were purified by preparative PAGE.

### Luciferase readthrough assay of tRNAs

Hep3B or CFBE41o- cells were seeded in 96-well cell culture plates at 1×10⁴ cells/well and grown in Dulbecco's Modified Essential Medium (DMEM) for Hep3B or Minimum Essential Medium (MEM) for CFBE41o- cells supplemented with 10% FBS and 2 mM L-glutamine (PAN Biotech). 16 to 24 hours later, Hep3B or CFBE41o-cells were co-transfected in triplicate with 25 ng PTC-FLuc or WT-FLuc plasmids and 100 ng each tRNA variant using lipofectamine 3000 (Thermo Fisher Scientific). After four to six hours, medium was replaced and 24 hours post-transfection cells were lysed with 1x passive lysis buffer (Promega) and luciferase activity measured with luciferase assay system (Promega) and Spark microplate reader (Tecan).

### Stimulation of hPBMCs with tRNAs

IFN-a levels of human peripheral blood mononuclear cells (hPBMCs) were determined as described in Kaiser et al. 2014 (Kaiser, S., Rimbach, K., Eigenbrod, T., Dalpke, A. H. & Helm, M. A modified dinucleotide motif specifies tRNA recognition by TLR7. RNA 20, 1351-1355 (2014). Briefly, hPBMCs were seeded in 96-well culture plate at 4×10⁵

## Claims

1. A synthetic transfer RNA comprising at least one modified nucleotide at a given position or at least one pair of modified nucleotides at given positions, the modified nucleotide or pair of modified nucleotides and their position or positions being selected from the following list:
a) m5C or 2'-OMeC at position 11,
b) m6A, m1A or 2'-OMeA at position 21,
c) Ψ, 2'-OMeΨ, m5U, m1Ψ, or 2'-OMeU at position 37
d) m5C or 2'-OMeC at position 56,
e) m6A, m1A or 2'-OMeA at position 59,
f) Ψ, 2'-OMeΨ, m5U, m1Ψ, or 2'-OMeU at position 60,
g) m5C, m6A, m5U, m1A, 2'-OMeA, 2'-OMeC, 2'-OMeU, 2'-OMeΨ, Ψ, or m1Ψ at position 73,
h) m6A, m1A or 2'-OMeA at position 49 and Ψ, 2'-OMeΨ, m5U, m1Ψ, or 2'-OMeU at position 65,
i) m6A, m1A or 2'-OMeA at position 65 and Ψ, 2'-OMeΨ, m5U, m1Ψ, or 2'-OMeU at position 49,
j) Ψ, 2'-OMeΨ, m5U, m1Ψ, or 2'-OMeU at position 2 and m6A, m1A or 2'-OMeA at position 71,
k) Ψ, 2'-OMeΨ, m5U, m1Ψ, or 2'-OMeU at position 71 and m6A, m1A or 2'-OMeA at position 2,
l) Ψ, 2'-OMeΨ, m5U, m1Ψ, or 2'-OMeU at position 5 and m6A, m1A or 2'-OMeA at position 68,
m) Ψ, 2'-OMeΨ, m5U, m1Ψ, or 2'-OMeU at position 68 and m6A, m1A or 2'-OMeA at position 5,
n) m6A, m1A or 2'-OMeA at position 29 and Ψ, 2'-OMeΨ, m5U, m1Ψ, or 2'-OMeU at position 41, or
o) m6A, m1A or 2'-OMeA at position 41 and Ψ, 2'-OMeΨ, m5U, m1Ψ, or 2'-OMeU at position 29,
the position numbering following transfer RNA numbering convention.

2. The synthetic transfer RNA according to claim 1, wherein the synthetic transfer RNA carries a three-base anticodon base-pairing with a stop codon on an mRNA, or carries an extended four-base, five-base or six-base anticodon.

3. The synthetic transfer RNA according to one of claims 1 or 2, wherein the transfer RNA includes at least one m5C nucleotide, preferably includes at least one additional modified nucleotide selected from m5C at position 72, m5C at position 38, m5C at position 48, m5C at position 49 and m5C at position 50, or a combination of any number of these modified nucleotides at these positions, the position numbering following transfer RNA numbering convention.

4. The synthetic transfer RNA according to one of the preceding claims, wherein the synthetic transfer RNA additionally has at least one, preferably at least two, more preferably at least three or most preferred all of the following modified nucleotides: D at position 16, D at position 17, 2'-OMeG at position 18, D at position 20, D at position 20a, Ψ at position 28, Ψ at position 30, Ψ at position 31, Ψ or 2'-OMeΨ at position 39, 2'-OMeU at position 44, m5C at position 48, Ψ or m5u at position 54, Ψ at position 55, the position numbering following transfer RNA numbering convention.

5. The synthetic transfer RNA according to one of the preceding claims, wherein the synthetic tRNA is not identical to any naturally occurring human tRNA.

6. The synthetic transfer RNA according to one of the preceding claims, wherein the synthetic tRNA comprises
a) an anticodon arm having or comprising one of the sequences of SEQ ID NO: 01-16 or one of the sequences of SEQ ID NO: 17-32, and/or
b) a variable loop having or comprising one of the sequence of SEQ ID NO: 33-34, and/or
c) a T arm having or comprising one of the sequences according to SEQ ID NO: 35-38.

7. The synthetic transfer RNA according to one of the preceding claims, wherein the synthetic tRNA does not include Cm at position 56, m1Ψ at position 54 or archaeosine (G^{∗}) at position 15, the position numbering following transfer RNA numbering convention.

8. The synthetic transfer RNA according to one of the preceding claims for use as a medicament.

9. The synthetic transfer RNA according to one of claims 1 to 7 for use as a medicament in a disease, which is at least partly caused by a nonsense or frameshift mutation leading to the production of a protein being dysfunctional or non-functional compared to the wild-type protein, or at least partly caused by the intracellular sequestration of a tRNA leading to depletion of the cellular pool of the tRNA.

10. The synthetic transfer RNA according to one of claims 1 to 7 for use as a medicament for treating Hurler syndrome, betha-thalassemia, Crohn's disease, Tay-Sachs disease, Duchenne muscular dystrophy, cystic fibrosis, neuronal ceroid lipofuscinosis, neurofibromatosis type 1, or Charcot-Marie-Tooth disease.

11. Pharmaceutical composition, comprising a synthetic transfer RNA according to one of claims 1 to 7, and a pharmaceutically acceptable carrier.
